# EUROPÄISCHE PATENTSCHRIFT

(11) **EP 1 880 669 B1**
(45) Veröffentlichungstag und Bekanntmachung des Hinweises auf die Patenterteilung: **25.03.2009**
(21) Anmeldenummer: 06015295.6
(22) Anmeldetag: 22.07.2006
(51) Int. Cl.: A61B 5/15, A61B 10/00, A61B 5/1473, G01N 33/483, F04B 43/12, G01N 33/487, A61B 5/145, A61M 1/00, A61M 1/36, B01D 19/00, A61M 1/34, A61M 1/16, B01D 69/08, G01N 1/14, G01N 1/20, A61M 5/142

(54) **Tragbare Messeinrichtung zur Bestimmung einer medizinisch bedeutsamen Analytkonzentration**
Portable medical device for measuring analyte concentration
Dispositiv médical de mesure d'une concentration d'analyte

(43) Veröffentlichungstag der Anmeldung: 23.01.2008
(73) Patentinhaber: Roche Diagnostics GmbH, 68305 Mannheim (DE); F.HOFFMANN-LA ROCHE AG, 4070 Basel (CH)
(72) Erfinder: Ocvirk, Gregor, Dr., 68239 Mannheim (DE); Kraemer, Peter, 67146 Deidesheim (DE)
(74) Vertreter: Mommer, Niels

(56) Entgegenhaltungen:
- EP-A- 0 187 109
- WO-A-00/22977
- WO-A2-02/055128
- US-A- 5 002 054
- US-A- 5 615 671
- US-A- 6 013 029

## Beschreibung

Die Erfindung betrifft eine tragbare Messeinrichtung zur Bestimmung einer Konzentration eines Analyten in einem lebenden Körper mit den im Oberbegriff des Anspruchs 1 angegebenen Merkmalen. Eine Messeinrichtung entsprechend dem Oberbegriff von Anspruch 1 ist aus der WO 00/22977 A bekannt, bei der Flüssigkeit zum Entfernen störender Gasblasen in eine Gasblasenfalle gesaugt wird.

Messeinrichtungen zur Bestimmung einer Konzentration eines Analyten in einem lebenden Körper sind im Stand der Technik insbesondere als so genannte Mikrodialysesysteme bekannt und in der US 2002/0082490 A1 beschrieben. Bei einem Mikrodialysesystem wird eine Perfusionsflüssigkeit durch eine in Körpergewebe implantierte Mikrodialysesonde hindurch gepumpt, so dass Analytmoleküle aus die Sonde umgebendem Körpergewebe und/oder Körperflüssigkeit in die Perfusionsflüssigkeit hinein diffundieren. Im Idealfall wird auf diese Weise erreicht, dass sich in der Perfusionsflüssigkeit dieselbe Analytkonzentration wie in dem die Sonde umgebenden Körpergewebe einstellt. Zur Bestimmung der Analytkonzentration in einer menschlichen oder tierischen Körperflüssigkeit wird anschließend mit einem Sensor des Mikrodialysesystems die Analytkonzentration in der Perfusionsflüssigkeit gemessen.

Wichtigste Anwendung von Mikrodialysesystemen ist die Messung der Glukosekonzentration zur Diabetesbehandlung. Die dafür verwendeten Messeinrichtungen müssen klein und leicht sein, da sie von Diabetikern ständig mitgeführt werden. Prinzipiell ist es zur kontinuierlichen Messung des Glukosegehalts mit einer tragbaren Messeinrichtung aber nicht erforderlich, dass eine Perfusionslösung durch eine Mikrodialysesonde hindurch gepumpt wird. Möglich ist es auch, mit einer Sonde kontinuierlich eine geringe Menge Körperflüssigkeit aus Körpergewebe zu entnehmen und mit einem Sensor die Konzentrationsbestimmung unmittelbar an der Körperflüssigkeit vorzunehmen.

Tragbare Messeinrichtung der eingangs genannten Art müssen einerseits möglichst klein und leicht sein, um von einem Patienten ständig mitgeführt werden zu können, andererseits eine medizinisch bedeutsame Analytkonzentration zuverlässig und mit hoher Präzision bestimmen.

Aufgabe der Erfindung ist es deshalb einen Weg aufzuzeigen, wie bei einer tragbaren Messeinrichtung der eingangs genannten Art die Messgenauigkeit verbessert werden kann, ohne Größe und Gewicht der Messeinrichtung wesentlich zu erhöhen.

Diese Aufgabe wird erfindungsgemäß mit einer tragbaren Messeinrichtung der eingangs genannten Art, dadurch gelöst, dass die Flüssigkeits-Fördereinrichtung eine Entgasungseinrichtung zum Entgasen der dem Sensor von der Sonde über die Flüssigkeitsleitung zuzuführenden Flüssigkeit umfasst.

Im Rahmen der Erfindung wurde festgestellt, dass die Messgenauigkeit durch Schwankungen des Gasgehalts der zu analysierenden Flüssigkeit, insbesondere durch darin auftretende Gasblasen, beeinträchtigt wird. Durch Entgasen der zu analysierenden Flüssigkeit lässt sich deshalb die Messgenauigkeit wesentlich steigern. Um eine bedeutende Verbesserung gegenüber dem Stand der Technik zu erzielen, ist es dabei nicht unbedingt erforderlich, dass die zu untersuchende Flüssigkeit vollständig entgast wird, da bereits durch das Entfernen von Gasblasen die Präzision und Zuverlässigkeit von Messergebnissen der Analytkonzentration erheblich gesteigert werden kann.

Um mit tragbaren Messeinrichtungen eine medizinisch bedeutsame Analytkonzentration bestimmen zu können, sind neben der eigentlichen Messeinrichtung verschiedene Verbrauchsmaterialen erforderlich, beispielsweise Sonden, die bestimmungsgemäß in Körpergewebe implantiert werden, und Schläuche zum Anschluss einer Sonde an die Flüssigkeitsleitung der Messeinrichtung. Derartige Verbrauchsmaterialen und eine dazugehörende Messeinrichtung bilden ein tragbares Messsystem zur Bestimmung einer Analytkonzentration. Ein weiterer Aspekt der Erfindung betrifft deshalb ein tragbares Messsystem zur Bestimmung einer medizinisch bedeutsamen Analytkonzentration mit einer erfindungsgemäßen Messeinrichtung, die einen Sensor und eine Flüssigkeitsfördereinrichtung umfasst, und eine in Körpergewebe implantierbare Sonde, die im Betrieb an die Flüssigkeitsfördereinrichtung angeschlossen ist.

Die Entgasungseinrichtung der erfindungsgemäßen Messeinrichtung umfasst eine Absaugleitung, mit der im Betrieb ein Unterdruck an eine gasdurchlässige Oberfläche der Flüssigkeitsleitung angelegt wird. Beispielsweise kann ein Abschnitt der Flüssigkeitsleitung als gasdurchlässige Hohlfaser ausgebildet werden, die in einer Entgasungskammer angeordnet ist, an welche die Absaugleitung angeschlossen ist. Besonders günstig ist es dabei, dass die Flüssigkeitsleitung einen als Schlauch ausgebildeten Abschnitt umfasst, auf den die Pumpe zum Erzeugen eines Förderdrucks peristaltisch einwirkt und die Absaugleitung ebenfalls einen als Schlauch ausgebildeten Abschnitt umfasst, auf den die Pumpe zum Erzeugen des Unterdrucks peristaltisch einwirkt. Auf diese Weise kann mit einer einzigen Pumpe, die für eine Flüssigkeitsfördereinrichtung ohnehin benötigt wird, sowohl der erforderliche Förderdruck als auch der Unterdruck erzeugt werden und mit einem kompakten Gerät eine Entgasung der zu analysierenden Flüssigkeit bewirkt werden.

Dokument EP0187109 offenbart eine Flüssigkeitsfördereinrichtung in einem Dialysegerät, in dem eine Peristaltikpumpe einen Druckunterschied an einer Membrane erzeugt, aber die Membrane keine gasdurchlässige Oberfläche besitzt und die Einrichtung nicht zum Entgasen ausgebildet ist.

Peristaltikpumpen sind aus anderen Bereichen der Technik bekannt und beispielsweise in der WO 2004/109109 A1 beschrieben. Mit einer Peristaltikpumpe lässt sich sowohl eine gleichmäßige Förderleistung, die für eine präzise Analyse von Bedeutung ist, als auch eine kompakte Entgasungseinrichtung verwirklichen. Ein weiterer Aspekt, der auch unabhängige Bedeutung hat, betrifft deshalb eine Flüssigkeitsfördereinrichtung nach Anspruch 9 zum Entgasen von Flüssigkeiten, umfassend eine Flüssigkeitsleitung zum Fördern der Flüssigkeit, eine Pumpe zum Erzeugen eines Förderdrucks in der Flüssigkeitsleitung, und eine Absaugleitung, mit der im Betrieb ein Unterdruck an eine gasdurchlässige Oberfläche der Flüssigkeitsleitung angelegt wird, wobei die Pumpe eine Peristaltikpumpe ist, die Flüssigkeitsleitung einen als Schlauch ausgebildeten Abschnitt umfasst, auf den die Pumpe im Betrieb zum Erzeugen des Förderdrucks peristaltisch einwirkt, und die Absaugleitung einen als Schlauch ausgebildeten Abschnitt umfasst, auf den die Pumpe im Betrieb zum Erzeugen des Unterdrucks peristaltisch einwirkt.

Weitere Einzelheiten und Vorteile der Erfindung werden im Folgenden im Rahmen von Ausführungsbeispielen unter Bezugnahme auf die beigefügten Zeichnungen erläutert, wobei gleiche und einander entsprechende Teile mit identischem Bezugszeichen gekennzeichnet sind. Die Merkmale der im Folgenden beschriebenen Ausführungsbeispiele können einzeln oder in Kombination zum Gegenstand von Ansprüchen gemacht werden. Es zeigen:
- Fig. 1: eine schematische Darstellung eines Ausführungsbeispiels einer erfindungsgemäßen Messeinrichtung;
- Fig. 2: eine schematische Darstellung eines weiteren Ausführungsbeispiels einer erfindungsgemäßen Messeinrichtung;
- Fig. 3: eine schematische Darstellung eines weiteren Ausführungsbeispiels einer erfindungsgemäßen Messeinrichtung; und
- Fig. 4: eine schematische Darstellung eines weiteren Ausführungsbeispiels einer erfindungsgemäßen Messeinrichtung.

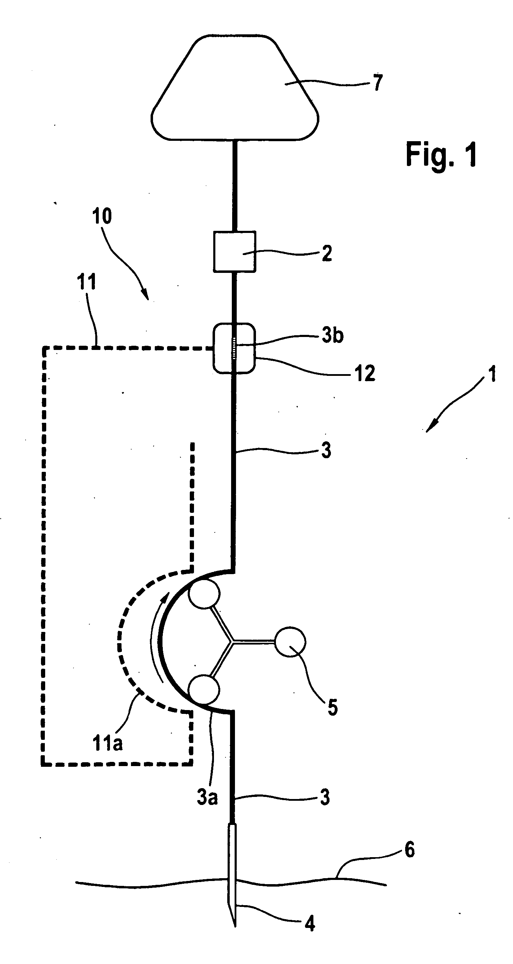

Fig. 1 zeigt eine schematische Darstellung eines Ausführungsbeispiels einer Messeinrichtung 1 zur Bestimmung einer Konzentration eines Analyten in einem lebenden Körper. Bei dem dargestellten Ausführungsbeispiel handelt es sich bei dem Analyten um Glukose. Die Messeinrichtung 1 umfasst einen Sensor 2 zur Bestimmung der Konzentration eines Analyten in einer Flüssigkeit und eine Flüssigkeitsfördereinrichtung mit einer Flüssigkeitsleitung 3 zum Anschließen an eine in Körpergewebe implantierbare Sonde 4 und einer Pumpe 5, um Flüssigkeit von der Sonde 4 durch die Flüssigkeitsleitung 3 zu dem Glucosesensor 2 zu pumpen. Die Messeinrichtung 1 bildet zusammen mit der Sonde 4, die in Figur 1 in einem menschlichen Körper 6 implantiert dargestellt ist, ein tragbares Messsystem, das stromnetzunabhängig ist. Bei dem Sensor 2 handelt es sich um einen elektrochemischen Sensor. Der Sensor 2 ist als Durchflusssensor ausgebildet.

Mit der Pumpe 5 wird bei dem in Fig. 1 dargestellten Ausführungsbeispiel eine Körperflüssigkeit, beispielsweise Blut, aus der Sonde 4 durch die Flüssigkeitsleitung 3 zu dem Sensor 2 gepumpt. Von dem Sensor 2 aus gelangt die untersuchte Flüssigkeit in einen Abfallbehälter 7, in dem sie bis zur Entsorgung aufbewahrt wird. Um die Messgenauigkeit des Sensors 2 zu erhöhen, enthält die Flüssigkeits-Fördereinrichtung eine Entgasungseinrichtung 10 zum Entgasen der dem Sensor 2 von der Sonde 4 über die Flüssigkeitsleitung 3 zuzuführenden Flüssigkeit. Die Entgasungseinrichtung 10 umfasst eine in Fig. 1 gestrichelt dargestellte Absaugleitung 11, mit der im Betrieb ein Unterdruck an eine gasdurchlässige Oberfläche der Flüssigkeitsleitung 3 angelegt wird. Die gasdurchlässige Oberfläche der Flüssigkeitsleitung ist in einer Entgasungskammer 12 angeordnet, an welche die Absaugleitung 11 angeschlossen ist. Die gasdurchlässige Oberfläche der Flüssigkeitsleitung 3 wird von einer Hohlfaser zur Verfügung gestellt, die einen Abschnitt der Flüssigkeitsleitung bildet, der in der Entgasungskammer 12 angeordnet ist. Geeignete Hohlfasern mit einer gasdurchlässigen, jedoch flüssigkeitsdichten Oberfläche werden unter der Bezeichnung Oxyphan bzw. Oxyplus von Membrana angeboten. Geeignete Materialen für derartige Hohlfasern sind insbesondere hydrophobe Kettenmoleküle, beispielsweise Polypropylen und Polymethylpenten.

Bei der Pumpe 5 handelt es sich um eine Peristaltikpumpe, mit der sowohl der Förderdruck der Flüssigkeitsleitung 3 als auch der Unterdruck der Absaugleitung 11 erzeugt wird. Die Flüssigkeitsleitung 3 umfasst einen als Schlauch ausgebildeten Abschnitt 3a, auf den die Pumpe im Betrieb zum Erzeugen des Förderdrucks peristaltisch einwirkt. Die Absaugleitung 11 umfasst einen als Schlauch ausgebildeten Abschnitt 11a, auf den die Pumpe 5 im Betrieb zum Erzeugen des Unterdrucks peristaltisch einwirkt.

Peristaltikpumpen bestehen im Wesentlichen aus einem Schlauchbett als Auflage für den Schlauch und mindestens einem Element, das den Schlauch in einem Teilbereich quetscht. Die Pumpwirkung wird erzeugt, in dem das Quetschelement relativ zum Schlauch in Längsrichtung bewegt wird. Als Quetschelemente sind Stößel, Finger oder Rollen auf einem Rotor bekannt. Bei dem dargestellten Ausführungsbeispiel sind drei Rollen dargestellt, die in Pfeilrichtung an dem Schlauch entlangbewegt werden. Die Schlauchabschnitte 3a, 11a der Flüssigkeitsleitung 3 und der Absaugleitung 11 sind nebeneinander auf einem Schlauchbett angeordnet, so dass die Quetschelemente der Peristaltikpumpe jeweils auf beide Schläuche gleichzeitig einwirken. Dabei ist es günstig, für die entsprechenden Abschnitte 3a, 11a der Flüssigkeitsleitung 3 und der Absaugleitung 11 gleiche Schläuche, insbesondere aus gleichem Material, beispielsweise PVC, und mit gleichem Außendurchmesser, zu verwenden.

Günstig ist es, wenn der als Schlauch ausgebildete erste Abschnitt 3a der Flüssigkeitsleitung 3, auf den die Pumpe 5 zum Erzeugen des Förderdrucks peristaltisch einwirkt, eine größere Wandstärke als ein in der Entgasungskammer 12 angeordneter zweiter Flüssigkeitsleitungsabschnitt 3b hat. Der erste Abschnitt 3a der Flüssigkeitsleitung 3, auf den die Pumpe 5 zum Erzeugen des Förderdrucks peristaltisch einwirkt, kann auf diese Weise widerstandsfähiger ausgebildet werden, so dass er der peristaltischen Einwirkung der Pumpe 5 lange Zeit ohne Verschleißerscheinungen standhalten kann. Der in der Entgasungskammer 12 angeordnete zweite Flüssigkeitsleitungsabschnitt 3b ist jedoch keiner derartigen Beanspruchung ausgesetzt, so dass er besonders dünnwandig ausgeführt werden kann, um die gasdurchlässige Oberfläche zur Verfügung zu stellen. Die beiden Abschnitte 3a, 3b der Flüssigkeitsleitung sind stoffschlüssig, beispielsweise durch kleben, miteinander verbunden. Bei der Dimensionierung des Hohlraumes der Entgasungskammer 12 und des darin verlaufenden zweiten Abschnitts 3b der Flüssigkeitsleitung 3 ist darauf zu achten, dass der Hohlraum der Entgasungskammer 12 einerseits lang genug ist, um einen zum Entfernen evtl. Gasblasen ausreichenden Gasdurchtritt zu ermöglichen, und andererseits möglichst kurz ist, damit sich bei Inbetriebnahme möglichst schnell der erforderliche Unterdruck in der Entgasungskammer 12 erzeugen lässt. Die optimale Länge der Entgasungskammer 12 hängt deshalb von der Fließgeschwindigkeit der Flüssigkeit in der Flüssigkeitsleitung 3 und vom Innendurchmesser des Schlauchabschnitts 11 ab. Günstig sind Förderleistungen und Flüssigkeits-Fördereinrichtung von 0,1 µl/min bis 1 µl/min, insbesondere 0,2 µl/min bis 0,4 µl/min. Ferner ist darauf zu achten, dass das Volumen des Hohlraumes der Entgasungskammer 12, das den Abschnitt 3b der Flüssigkeitsleitung 3 umgibt, so groß ausgelegt ist, dass im Betrieb der relative Feuchtegehalt im Hohlraum gering gehalten wird und der erforderliche Unterdruck aufrecht erhalten werden kann.

Der in der Entgasungskammer 12 angeordnete zweite Flüssigkeitsleitungsabschnitt 3b hat bevorzugt eine Wandstärke von weniger als 0,2 mm, insbesondere weniger als 0,1 mm. Der als Schlauch ausgebildete erste Abschnitt 3a der Flüssigkeitsleitung 3, auf den die Pumpe 5 zum Erzeugen des Förderdrucks peristaltisch einwirkt, hat bevorzugt eine Wandstärke von mindestens 0,5 mm, insbesondere eine Wandstärke von 0,7 mm bis 2 mm. Der Innendurchmesser des Schlauchs, auf den die Pumpe 5 peristaltisch einwirkt, beträgt bevorzugt 0,5 mm bis 0,3 mm. Der in der Entgasungskammer 12 angeordnete zweite Abschnitt der Flüssigkeitsleitung hat bevorzugt einen Innendurchmesser von 0,1 mm bis 0,4 mm, insbesondere 0,2 mm bis 0,4 mm. Die Entgasungskammer 12 hat bevorzugt eine Länge, die das 5- bis 50-fache des Innendurchmessers der in der Entgasungskammer 12 verlaufenden Abschnitts der Flüssigkeitsleitung 3 beträgt. Günstig ist ein Volumen des Hohlraums der Entgasungskammer 12, der das 1,2 bis 1200-fache des Volumens beträgt, welches im Hohlraum durch den darin angeordneten zweiten Abschnitt 3b der Flüssigkeitsleitung 3 eingenommen wird. Die Entgasungskammer 12 ist bevorzugt als T-Stück aus Kunststoff ausgebildet, so dass die Flüssigkeitsleitung 3 mit geringem Aufwand durch die Entgasungskammer 12 hindurchgeführt und die Absaugleitung 11 an die Entgasungskammer 12 angeschlossen werden kann.

Damit die dargestellte Messeinrichtung von einem Benutzer ständig mitgeführt werden kann, ist sie netzunabhängig betreibbar und hat deshalb ein Aufnahmefach (nicht dargestellt) für Batterien. Die Masse der Messeinrichtung beträgt weniger als 1 kg, so dass ein Benutzer durch deren Gewicht nur wenig belastet ist.

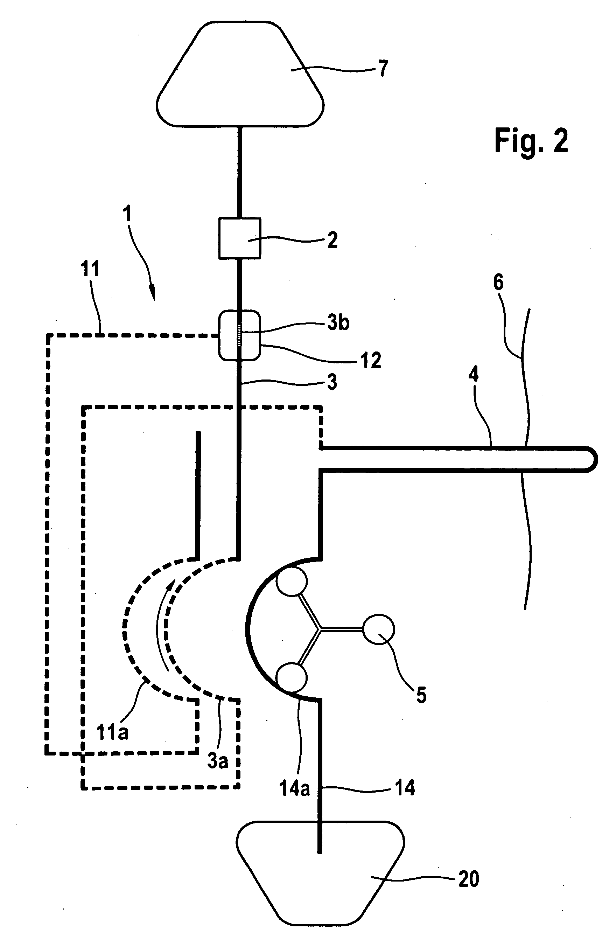

In Fig. 2 ist schematisch ein weiteres Ausführungsbeispiel eines Messsystems dargestellt, das eine Messeinrichtung 1 und eine Sonde 4 umfasst, die in Fig. 2 in einen lebenden Körper 6 implantiert dargestellt ist. Während bei dem anhand von Fig. 1 erläuterten Ausführungsbeispiel mit der Sonde 4 eine Körperflüssigkeitsprobe 1 entnommen und mittels des Sensors 2 untersucht wurde, wird bei dem in Fig. 2 dargestellten Messsystem eine Perfusionsflüssigkeit durch die als Mikrodialysesonde ausgebildete Sonde 4 hindurchgeleitet. Die Mikrodialysesonde 4 hat eine Dialysemembran, durch die Analytmoleküle aus die Sonde 4 umgebender Körperflüssigkeit in die Perfusionsflüssigkeit diffundieren können, so dass die Perfusionsflüssigkeit anschließend zur Bestimmung der Analytkonzentration durch die Flüssigkeitsleitung 3 zu dem Sensor 2 geleitet werden kann. Bei dem dargestellten Messsystem handelt es sich also um ein Mikrodialysesystem.

Die in Fig. 2 dargestellte Messeinrichtung 1 hat eine Perfusionsflüssigkeitsleitung 14, die dazu dient, Perfusionsflüssigkeit aus einem Perfusionsflüssigkeitsreservoir 20 in die Mikrodialysesonde 4 zu leiten. Ein Abschnitt 14a der Perfusionsflüssigkeitsleitung 14 ist als Schlauch ausgebildet, auf den die Peristaltikpumpe 5 zum Erzeugen eines Förderdrucks peristaltisch einwirkt. Dieser Schlauchabschnitt 14a ist in dem Schlauchbett der Pumpe 5 neben den Schläuchen 3a, 11a der Absaugleitung 11 und der Flüssigkeitsleitung 3 angeordnet, so dass die Quetschelemente der Peristaltikpumpe 5 auf alle 3 Schläuche 3a, 11a, 14a gemeinsam einwirken.

Geeignete Dialysesonden 4 und weitere Einzelheiten des Mikrodialysesystems sind in der US 2002/0082490 A1 offenbart, die diesbezüglich durch Bezugnahme zum Gegenstand der vorliegenden Anmeldung gemacht wird. Der einzig wesentliche Unterschied zwischen dem in Fig. 2 dargestellten Messsystem und dem aus der US 2002/0082490 A1 bekannten Mikrodialysesystem besteht darin, dass bei dem dargestellten Ausführungsbeispiel eine Flüssigkeitsfördereinrichtung mit einer Entgasungseinrichtung 11, 12 hinzugefügt wurde, um die Zuverlässigkeit und Genauigkeit der mit dem Sensor 2 gewonnenen Informationen über die Analytkonzentration zu erhöhen.

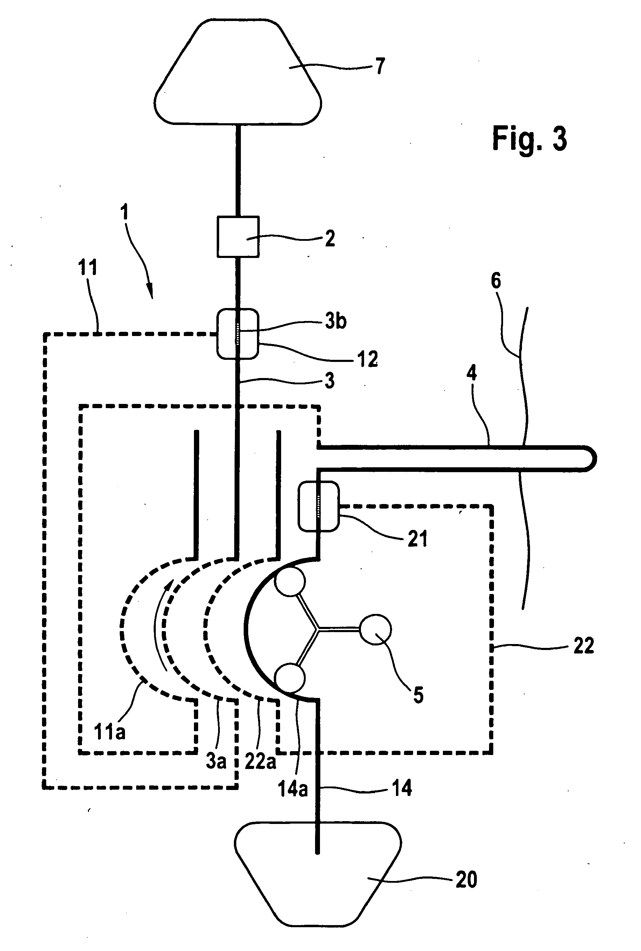

In Fig. 3 ist schematisch ein weiteres Ausführungsbeispiel eines Mikrodialysesystems dargestellt. Das in Fig. 3 dargestellte Mikrodialysesystem unterscheidet sich von dem in Fig. 2 dargestellten Mikrodialysesystem dadurch, dass eine zweite Entgasungseinrichtung mit einer zweiten Entgasungskammer 21 zum Entgasen der in die Sonde 4 einzuleitenden Perfusionsflüssigkeit. Bei dem dargestellten zweiten Ausführungsbeispiel verläuft die Perfusionsleitung 14 durch die zweite Entgasungskammer 21, an die eine zweite Unterdruckleitung 22 angelegt ist. Die Unterdruckleitung 22 hat ebenfalls einen als Schlauch ausgebildeten Abschnitt 22a, auf den die Peristaltikpumpe 5 zum Erzeugen des Unterdrucks peristaltisch einwirkt. Der in der zweiten Entgasungskammer 21 verlaufende Abschnitt 14a der Perfusionsflüssigkeitsleitung 14 hat ebenso wie der entsprechende in der ersten Entgasungskammer 12 verlaufende zweite Abschnitt 3a der Flüssigkeitsleitung 3 eine gasdurchlässige Oberfläche und kann beispielsweise als Hohlfaser ausgebildet sein.

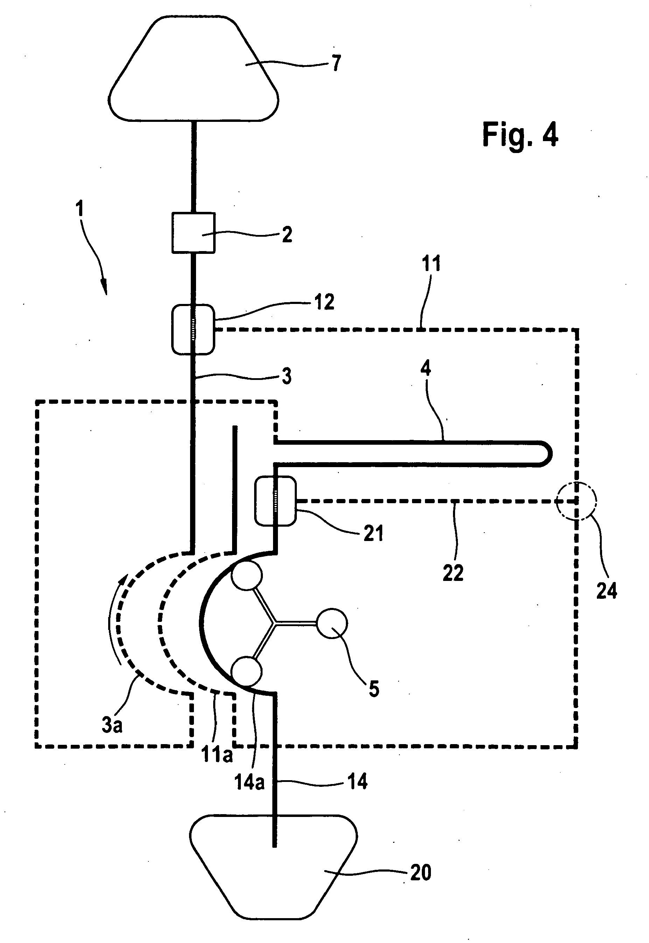

In Fig. 4 ist schematisch ein weiteres Ausführungsbeispiel eines Mikrodialysesystems dargestellt. Das in Fig. 4 dargestellte Mikrodialysesystem unterscheidet sich von dem in Fig. 3 dargestellten Mikrodialysesystem dadurch, dass die Unterdruckleitung 11 und die Unterdruckleitung 22 miteinander mit einem T-Stück 24 verbunden sind, so dass lediglich ein als Schlauch ausgebildeter Abschnitt 11a erforderlich ist, auf den die Peristaltikpumpe 5 zum Erzeugen des Unterdrucks peristaltisch einwirkt. Gegenüber Fig. 3 ergibt sich dadurch die Möglichkeit das erforderliche Moment der Peristaltikpumpe 5 und somit deren Stromaufnahme zu reduzieren.

### Bezugszeichenliste

- 1: Tragbare Messeinrichtung
- 2: Sensor
- 3: Flüssigkeitsleitung
- 3a: erster Abschnitt der Flüssigkeitsleitung
- 3b: zweiter Abschnitt der Flüssigkeitsleitung
- 4: Sonde
- 5: Peristaltikpumpe
- 6: Hautoberfläche
- 7: Abfallbehälter
- 10: Entgasungseinrichtung
- 11: Absaugleitung
- 11a: Abschnitt der Absaugleitung
- 12: Entgasungskammer
- 14: Perfusionsflüssigkeitsleitung
- 14a: Abschnitt der Perfusionsflüssigkeitsleitung
- 20: Perfusionsflüssigkeitsreservoir
- 21: Entgasungskammer
- 22: Absaugleitung
- 22a: Abschnitt der Unterdruckleitung 22
- 24: T-Stück

## Patentansprüche

1. Tragbare Messeinrichtung zur Bestimmung einer Konzentration eines Analyten in einem lebenden Körper umfassend
einen Sensor (2) zur Bestimmung der Konzentration eines in einer Flüssigkeit vorhandenen Analyten und
eine Flüssigkeits-Fördereinrichtung mit
einer Flüssigkeitsleitung (3) zum Anschließen an eine in Körpergewebe implantierbare Sonde (4),
einer Pumpe (5), um Flüssigkeit von der Sonde (4) durch die Flüssigkeitsleitung (3) zu dem Sensor (2) zu pumpen, und
einer Entgasungseinrichtung (10) zum Entgasen der dem Sensor (2) von der Sonde (4) über die Flüssigkeitsleitung (3) zuzuführenden Flüssigkeit, wobei die Entgasungseinrichtung (10) eine Absaugleitung (11) umfasst, mit der im Betrieb ein Unterdruck an eine gasdurchlässige Oberfläche (3b) der Flüssigkeitsleitung (3) angelegt wird,
**dadurch gekennzeichnet, dass**
die Pumpe (5) eine Peristaltikpumpe ist,
die Flüssigkeitsleitung (3) einen als Schlauch ausgebildeten Abschnitt (3a) umfasst, auf den die Pumpe (5) im Betrieb zum Erzeugen eines Förderdrucks peristaltisch einwirkt, und
die Absaugleitung (11) einen als Schlauch ausgebildeten Abschnitt (11a) umfasst, auf den die Pumpe (5) im Betrieb zum Erzeugen des Unterdrucks peristaltisch einwirkt.

2. Messeinrichtung nach Anspruch 1**, dadurch gekennzeichnet, dass** die Messeinrichtung (4) eine Masse von weniger als 1 kg hat.

3. Messeinrichtung nach einem der vorstehenden Ansprüche, **dadurch gekennzeichnet, dass** die gasdurchlässige Oberfläche der Flüssigkeitsleitung (3) in einer Entgasungskammer (12) angeordnet ist, an welche die Absaugleitung (11) angeschlossen ist.

4. Messeinrichtung nach einem der vorstehenden Ansprüche, **dadurch gekennzeichnet, dass** der als Schlauch ausgebildete erste Abschnitt (3a) der Flüssigkeitsleitung (3), auf den die Pumpe (5) zum Erzeugen des Förderdrucks peristaltisch einwirkt, eine größere Wandstärke als ein in der Entgasungskammer (12) angeordneter zweiter Flüssigkeitsleitungsabschnitt (3b) mit der gasdurchlässigen Oberfläche hat.

5. Messeinrichtung nach Anspruch 4, **dadurch gekennzeichnet, dass** der zweite Abschnitt (3b) der Flüssigkeitsleitung (3) als Hohlfaser ausgebildet ist.

6. Messeinrichtung nach einem der Ansprüche 4 oder 5, **dadurch gekennzeichnet, dass** die Länge der Entgasungskammer das 5- bis 20-fache des Innendurchmessers des in der Entgasungskammer (12) verlaufenden Abschnitts (3b) der Flüssigkeitsleitung (3) beträgt.

7. Tragbares Messsystem zur Bestimmung einer medizinisch bedeutsamen Analytkonzentration, umfassend
eine Messeinrichtung (1) nach einem der vorhergehenden Ansprüche, die einen Sensor (2) und eine Flüssigkeits-Fördereinrichtung umfasst, und
eine in Körpergewebe implantierbare Sonde (4), die im Betrieb an die Flüssigkeitsfördereinrichtung angeschlossen ist.

8. Messsystem nach Anspruch 7, **gekennzeichnet durch** ein Perfusionsflüssigkeitsreservoir (20) und **dadurch gekennzeichnet, dass** die Messeinrichtung (1) eine Mikrodialyseeinrichtung mit einer Perfusionsflüssigkeitsleitung (14) ist, die dazu dient, Perfusionsflüssigkeit aus dem Perfusionsflüssigkeitsreservoir (20) in eine als Mikrodialysesonde ausgebildete Sonde (4) zu leiten, wobei die Sonde (4) eine Dialysemembran aufweist, **durch** die Analytmoleküle aus die Sonde (4) umgebender Körperflüssigkeit in die Perfusionsflüssigkeit diffundieren können, so dass die Perfusionsflüssigkeit anschließend zur Bestimmung der Analytkonzentration **durch** die Flüssigkeitsleitung (3) zu dem Sensor (2) geleitet werden kann.

9. Flüssigkeits-Fördereinrichtung zum Entgasen von Flüssigkeiten, insbesondere für eine Messeinrichtung (1) gemäß einem der Ansprüche 1 bis 7, umfassend
eine Flüssigkeitsleitung (3) zum Fördern der Flüssigkeit,
eine Pumpe (5) zum Erzeugen eines Förderdrucks in der Flüssigkeitsleitung (3), und
eine Absaugleitung (11), mit der im Betrieb ein Unterdruck an eine gasdurchlässige Oberfläche (3b) der Flüssigkeitsleitung (3) angelegt wird,
wobei
die Pumpe (5) eine Peristaltikpumpe ist,
die Flüssigkeitsleitung (3) einen als Schlauch ausgebildeten Abschnitt (3a) umfasst, auf den die Pumpe (5) im Betrieb zum Erzeugen des Förderdrucks peristaltisch einwirkt, und
die Absaugleitung (11) einen als Schlauch ausgebildeten Abschnitt (11a) umfasst, auf den die Pumpe (5) im Betrieb zum Erzeugen des Unterdrucks peristaltisch einwirkt.

## Claims

1. Portable measuring facility for determining the concentration of an analyte in a living body, comprising
a sensor (2) for determining the concentration of an analyte that is present in a liquid and
a liquid conveying facility having
a liquid line (3) for connection to a probe (4) that can be implanted into body tissue, and
a pump (5) in order to pump liquid from the probe (4) through the liquid line (3) to the sensor (2),
a degassing facility (10) for degassing the liquid that is to be guided from the probe (4) through the liquid line (3) to the sensor (2), wherein the degassing facility (10) comprises an aspiration line (11) that is used, in operation, to apply a negative pressure to a gas-permeable surface (3b) of the liquid line (3),
**characterized in that**
the pump (5) is a peristaltic pump,
the liquid line (3) comprises a section (3a) that is provided in the form of a tube on which the pump (5), in operation, acts in a peristaltic fashion in order to generate a conveying pressure, and
the aspiration line (11) comprises a section (11a) that is provided in the form of a tube on which the pump (5), in operation, acts in a peristaltic fashion in order to generate the negative pressure.

2. Measuring facility according to claim 1, **characterized in that** the mass of the measuring facility (4) is less than 1 kg.

3. Measuring facility according to any one of the preceding claims, **characterized in that** the gas-permeable surface of the liquid line (3) is arranged within a degassing chamber (12) to which the aspiration line (11) is connected.

4. Measuring facility according to any one of the preceding claims, **characterized in that** the wall thickness of the first section (3a) of the liquid line (3) that is provided in the form of a tube and on which the pump (5) acts in a peristaltic fashion in order to generate the conveying pressure is larger than that of a second liquid line section (3b) having the gas-permeable surface that is arranged within the degassing chamber (12).

5. Measuring facility according to claim 4, **characterized in that** the second section (3b) of the liquid line (3) is provided in the form of a hollow fiber.

6. Measuring facility according to claim 4 or 5, **characterized in that** the length of the degassing chamber (12) is 5- to 20-fold the internal diameter of the section (3b) of the liquid line (3) that extends within the degassing chamber (12).

7. Portable measuring system for determining a medically-significant analyte concentration, comprising
a measuring facility (1) according to any one of the preceding claims that comprises a sensor (2) and a liquid conveying facility, and
a probe (4) that can be implanted in body tissue and, in operation, is connected to the liquid conveying facility.

8. Measuring system according to claim 7, **characterized by** a perfusion liquid reservoir (20) and in that the measuring facility (1) is a micro-dialysis facility having a perfusion liquid line (14) that serves to guide perfusion liquid from the perfusion liquid reservoir (20) into a probe (4) that is provided in the form of a micro-dialysis probe and comprises a dialysis membrane through which analyte molecules from body fluid surrounding the probe (4) can diffuse into the perfusion liquid such that the perfusion liquid can subsequently be guided through the liquid line (3) to the sensor (2) for determining the analyte concentration.

9. Liquid conveying facility for the degassing of liquids, in particular for a measuring facility (1) according to any one of the claims 1 to 7, comprising
a liquid line (3) for conveying the liquid,
a pump (5) for generating a conveying pressure in the liquid line (3), and
an aspiration line (11) that is used, in operation, to apply a negative pressure to a gas-permeable surface (3b) of the liquid line (3),
wherein
the pump (5) is a peristaltic pump,
the liquid line (3) comprises a section (3a) that is provided in the form of a tube on which the pump (5), in operation, acts in a peristaltic fashion in order to generate the conveying pressure, and
the aspiration line (11) comprises a section (11a) that is provided in the form of a tube on which the pump (5), in operation, acts in a peristaltic fashion in order to generate the negative pressure.

## Revendications

1. Dispositif de mesure portable pour déterminer une concentration d'une substance à analyser dans un corps vivant, comprenant
➢ un capteur (2) pour déterminer la concentration d'une substance à analyser présente dans un liquide, et
➢ un dispositif de refoulement de liquide, comprenant
➢ une conduite de liquide (3) à raccorder à une sonde (4) implantable dans un tissu corporel,
➢ une pompe (5) pour pomper du liquide de la sonde (4) à travers la conduite de liquide (3) jusqu'au capteur (2), et
➢ un dispositif de dégazage (10) pour dégazer le liquide à amener au capteur (2) par la sonde (4) par l'intermédiaire de la conduite de liquide (3), dans lequel le dispositif de dégazage (10) comprend une conduite d'aspiration (11) avec laquelle en cours de fonctionnement une dépressurisation est appliqué à une surface perméable au gaz (3b) de la conduite de liquide (3),
**caractérisé en ce que**
➢ la pompe (5) est une pompe péristaltique,
➢ la conduite de liquide (3) comprend un tronçon (3a) réalisé comme un tuyau sur lequel la pompe (5) agit de façon péristaltique en cours de fonctionnement pour la génération d'une pression de refoulement, et
➢ la conduite d'aspiration (11) comprend un tronçon (11a) réalisé comme un tuyau sur lequel la pompe (5) agit de façon péristaltique en cours de fonctionnement pour la génération de la dépressurisation.

2. Dispositif de mesure selon la revendication 1, **caractérisé en ce que** le dispositif de mesure (4) présente une masse de moins de 1 kg.

3. Dispositif de mesure selon l'une quelconque des revendications précédentes, **caractérisé en ce que** la surface perméable au gaz de la conduite de liquide (3) est disposée dans une chambre de dégazage (12) à laquelle la conduite d'aspiration (11) est raccordée.

4. Dispositif de mesure selon l'une quelconque des revendications précédentes, **caractérisé en ce que** le premier tronçon (3a) réalisé comme un tuyau de la conduite de liquide (3), sur lequel la pompe (5) agit de façon péristaltique pour générer la pression de refoulement, présente une épaisseur de paroi supérieure à un deuxième tronçon de conduite de liquide (3b) avec la surface perméable au gaz et disposé dans la chambre de dégazage (12).

5. Dispositif selon la revendication 4, **caractérisé en ce que** le deuxième tronçon (3b) de la conduite de liquide (3) est réalisé comme une fibre creuse.

6. Dispositif selon l'une quelconque des revendications 4 ou 5, **caractérisé en ce que** la longueur de la chambre de dégazage mesure de 5 à 20 fois le diamètre intérieur du tronçon (3b) de la conduite de liquide (3) s'étendant dans la chambre de dégazage (12).

7. Système de mesure portable pour déterminer une concentration de substance à analyser ayant une signification médicale, comprenant
➢ un dispositif de mesure (1) selon l'une quelconque des revendications précédentes qui comprend un capteur (2) et un dispositif de refoulement de liquide, et
➢ une première sonde (4) implantable dans un tissu corporel qui est raccordée en cours de fonctionnement au dispositif de refoulement de liquide.

8. Système de mesure selon la revendication 7, **caractérisé par** un réservoir de liquide de perfusion (20) et **caractérisé en ce que** le dispositif de mesure (1) est un dispositif de microdialyse avec une conduite de liquide de perfusion (14) qui sert à diriger du liquide de perfusion du réservoir de liquide de perfusion (20) dans une sonde (4) réalisée comme une sonde de microdialyse, dans lequel la sonde (4) présente une membrane de dialyse à travers laquelle des molécules d'une substance à analyser peuvent se diffuser à partir d'un liquide corporel entourant la sonde (4) dans le liquide de perfusion de sorte que le liquide de perfusion peut ensuite être dirigé à travers la conduite de liquide (3) jusqu'au capteur (2) pour la détermination de la concentration en substance à analyser.

9. Dispositif de refoulement de liquide pour le dégazage de liquides, en particulier pour un dispositif de mesure (1) selon l'une quelconque des revendications 1 à 7, comprenant
➢ une conduite de liquide (3) pour refouler le liquide,
➢ une pompe (5) pour générer une pression de refoulement dans la conduite de liquide (3), et
➢ une conduite d'aspiration (11) par laquelle en cours de fonctionnement une dépressurisation est appliqué à la surface perméable au gaz (3b) de la conduite de liquide (3),
dans lequel
➢ la pompe (5) est une pompe péristaltique,
➢ la conduite de liquide (3) comprend un tronçon (3a) réalisé comme un tuyau sur lequel agit la pompe (5) en cours de fonctionnement pour générer la pression de refoulement, et
➢ la conduite d'aspiration (11) comprend un tronçon (11a) réalisé comme un tuyau sur lequel agit la pompe (5) en cours de fonctionnement pour générer la dépressurisation.
